# EUROPÄISCHE PATENTSCHRIFT

(11) **EP 2 114 844 B1**
(45) Veröffentlichungstag und Bekanntmachung des Hinweises auf die Patenterteilung: **29.09.2010**
(21) Anmeldenummer: 08708209.5
(22) Anmeldetag: 25.01.2008
(51) Int. Cl.: C07C 29/17, C07C 45/50, C07C 45/74, C07C 47/21, C07C 201/02, C07C 203/04, C10L 1/22

(54) **VERZWEIGTE DECYLNITRATE UND IHRE VERWENDUNG ALS VERBRENNUNGSVERBESSERER UND/ODER CETANZAHLVERBESSERER IN KRAFTSTOFFEN**
BRANCHED DECYL NITRATES AND THE USE THEREOF AS COMBUSTION IMPROVERS AND/OR CETANE NUMBER IMPROVERS IN FUELS
NITRATES DE DÉCYLE RAMIFIÉS ET LEUR UTILISATION EN TANT QU'AMÉLIORATEURS DE COMBUSTION ET/OU AMÉLIORATEURS D'INDICE DE CÉTANE DANS DES CARBURANTS

(30) Priorität: 29.01.2007 EP 07101307
(43) Veröffentlichungstag der Anmeldung: 11.11.2009
(73) Patentinhaber: BASF SE, 67056 Ludwigshafen (DE)
(72) Erfinder: KORMANN, Claudius, 67105 Schifferstadt (DE); REBHOLZ, Uwe, 67678 Mehlingen (DE); NORDMANN, Gero, Charlotte, NC 28277 (US); KARL, Jörn, Beijing 100020 (CN); KARRER, Lothar, 64319 Pfungstadt (DE)
(86) Internationale Anmeldenummer: PCT/EP2008/050883
(87) Internationale Veröffentlichungsnummer: WO 2008/092809

(56) Entgegenhaltungen:
- US-A- 2 280 217
- US-A- 4 426 542
- US-A- 5 162 568

## Beschreibung

Die vorliegende Erfindung betrifft spezielle verzweigte Decylnitrate sowie eine Mischung hieraus, die Herstellung dieser verzweigten Decylnitrate und dieser Mischung, weiterhin einen Kraftstoff und ein Kraftstoffadditiv-Konzentrat, welche diese verzweigten Decylnitrate oder diese Mischung enthalten, sowie die Verwendung dieser verzweigten Decylnitrate oder dieser Mischung als Verbrennungsverbesserer und/oder Cetanzahlverbesserer in Kraftstoffen.

Organische Nitrate sind seit langem als Zündbeschleuniger in Kraftstoffen, insbesondere in Mitteldestillaten, bekannt. Organische Nitrate werden auch seit langem zur Erhöhung der Cetanzahl in Dieselkraftstoffen verwendet. Höhere Cetanzahlen führen zu schnelleren Motorstarts, speziell bei kaltem Wetter, zu leiseren Motorgeräuschen, zu vollständigerer Verbrennung, zu weniger Rauchentwicklung und unter Umständen zu weniger Injektorverkokung.

Typische organische Nitrate, die sich als Verbrennungsverbesserer in Ottokraftstoffen oder als Cetanzahlverbesserer in Dieselkraftstoffen eignen, sind Nitrate kurz- und mittelkettiger linearer und verzweigter Alkanole und Nitrate von Cycloalkanolen wie n-Hexylnitrat, 2-Ethylhexylnitrat, n-Heptylnitrat, n-Octylnitrat, iso-Octylnitrat, sec.-Octylnitrat, n-Nonylnitrat, n-Decylnitrat, n-Dodecylnitrat, Cyclopentylnitrat, Cyclohexylnitrat, Methylcyclohexylnitrat und Isopropylcyclohexylnitrat. Kommerziell am bedeutsamsten hiervon ist das 2-Ethylhexylnitrat. Die genannten organischen Nitrate und ihre Anwendung als Verbrennungsverbesserer bzw. Cetanzahlverbesserer werden beispielsweise in den Dokumenten US 6 676 715 B2, US-A 4 473 378, US 2003/0110684 A1, US 7 018 433 B2, US-A 5 782 937 und US 7 029 506 B2 beschrieben.

Desweiteren offenbart US 2 280 217 in Tabelle 2 die Cetanzahlen einer Reihe von Alkylnitraten, welche in unerwarteter Weise ab dem decylnitrat wieder zunehmen.

In der US-A 4 479 905 wird die Herstellung von organischen Nitraten durch Nitrierung einer Mischung aus aliphatischen primären Alkoholen, z.B. n-Octanol, 2-Ethylhexanol, n-Decanol oder 2-Ethyloctanol, und Alkoxyalkanolen, z.B. 2-Ethoxyethanol oder 2-Butoxyethanol, mittels Salpetersäure und Schwefelsäure beschrieben. Die so hergestellten Nitrat-Gemische eignen sich als Cetanzahlverbesserer in Dieselkraftstoffen.

Die im Stand der Technik als Verbrennungsverbesserer und/oder Cetanzahlverbesserer beschriebenen organischen Nitrate weisen eine Reihe von Nachteilen auf, insbesondere mangelnde thermische Stabilität, zu hohe Flüchtigkeit und nicht ausreichende Wirksamkeit. Eine zu hohe Flüchtigkeit als Folge eines niedrigeren Siedepunktes, welche naturgemäß bei Verbindungen mit niedrigerem Molekulargewicht auftritt, wirkt sich nachteilig auf Geruch, auf Flammpunkt und damit auf das mit solchen Verbindungen verbundene Sicherheitsrisiko im Umgang mit ihnen aus. Die Erfahrung des einschlägigen Fachmannes lehrt, dass mit höheren Molekulargewichten erwartungsgemäß zwar die Flüchtigkeit sinkt, jedoch auch die Wirksamkeit als Verbrennungsverbesserer und/oder Cetanzahlverbesserer nachlässt.

Die im Stand der Technik vorgeschlagenen Lösungen zur Behebung der Thermostabilitäts-Probleme wie die Mischung von hochwirksamen aber auch besonders explosionsempfindlichen organischen Nitraten wie C₁-C₃-Alkylnitraten mit höhermolekularen aber weniger wirksamen Nitraten wie Octylnitraten (beschrieben in der US-A 4 473 378) oder wie die Stabilisierung von Cetanverbesserern mit heterocyclischen Verbindungen mit längerkettigen Kohlenwasserstoffgruppen (beschrieben in der US 6 676 715 B2) können auch nicht überzeugen.

Es bestand daher die Aufgabe, organische Nitrate bereitzustellen, welche eine bessere oder zumindest die gleiche Wirksamkeit als Verbrennungsverbesserer oder Cetanzahlverbesserer wie der Marktstandard 2-Ethylhexylnitrat aufweisen, darüber hinaus jedoch eine geringere Flüchtigkeit, einen höheren Flammpunkt und eine höhere Temperatur der autokatalytischen Zersetzung, d.h. eine bessere Thermostabilität, als dieser besitzen. Weiterhin ist auch eine Verringerung des prozentualen Stickstoffgehaltes der bereitzustellenden organischen Nitrate erstrebenswert, um den Gehalt von Stickoxiden in den Auspuffgasen gering zu halten. Nicht zuletzt ist auch eine gute Tieftemperatur-Performance anzustreben.

Überraschenderweise wurde gefunden, dass spezielle verzweigte Decylnitrate diese Aufgabe erfüllen.

Das Nitrat aus 2-Ethyloctanol als verzweigtem Decanol ist bereits aus der US-A 4 479 905 bekannt. Da die aufgefundenen speziellen verzweigten Decylnitrate jedoch neue Substanzen darstellen, sind Gegenstand der vorliegenden Erfindung verzweigte Decylnitrate der Formel I

R¹R²CH-CH₂-O-NO₂ (I)

in der R¹ einen n-Propyl- oder iso-Propylrest bezeichnet und R² einen linearen oder verzweigten Alkylrest mit 5 Kohlenstoffatomen bedeutet.

Als Alkylrest mit 5 Kohlenstoffatomen für R² kann beispielsweise n-Pentyl, 1-Methylbutyl, 2-Methylbutyl, 3-Methylbutyl, 1-Ethylpropyl, 1,1-Dimethylpropyl, 2,2-Dimethylpropyl oder 1,2-Dimethylpropyl auftreten.

Typische verzweigte Decylnitrate der Formel I sind:
(Ia) 2-n-Propylheptylnitrat (R¹ = n-Propyl, R² = n-Pentyl)
(Ib) 2-iso-Propylheptylnitrat (R¹ = iso-Propyl, R² = n-Pentyl)
(Ic) 2-n-Propyl-4-methylhexylnitrat (R¹ = n-Propyl, R² = 2-Methylbutyl)
(Id) 2-iso-Propyl-4-methylhexylnitrat (R¹ = iso-Propyl, R² = 2-Methylbutyl)
(Ie) 2-n-Propyl-5-methylhexylnitrat (R¹ = n-Propyl, R² = 3-Methylbutyl)
(If) 2-iso-Propyl-5-methylhexylnitrat (R¹ = iso-Propyl, R² = 3-Methylbutyl)
(Ig) 2-n-Propyl-4,4-dimethylpentylnitrat (R¹ = n-Propyl, R² = 2,2-Dimethylpropyl)
(Ih) 2-iso-Propyl-4,4-dimethylpentylnitrat (R¹ = iso-Propyl, R² = 2,2-Dimethylpropyl)

Die Herstellung der genannten verzweigten Decylnitrate der Formel I erfolgt zweckmäßigerweise durch Nitrierung der entsprechenden verzweigten Decanole mittels eines Gemisches aus Salpetersäure und Schwefelsäure (üblicherweise als "Nitriersäure" bezeichnet). Man arbeitet vorzugsweise mit einem Gemisch aus ca. 65 gew.-%iger Salpetersäure und ca. 96 gew.-%iger Schwefelsäure im Vol.-Verhältnis von ca. 2:3 als Nitriersäure. Die Nitriersäure wird üblicherweise in hohem Überschuß gegenüber dem zu nitrierenden verzweigten Decanol eingesetzt, beispielweise im Mol-Verhältnis HNO₃ zu Decanol von 10:1 bis 25:1. Die Nitrierungsreaktion wird in der Regel bei Temperaturen von -10 bis + 25°C, vorzugsweise bei 0 bis +15°C unter ausreichender Kühlung durchgeführt. Wegen der starken Exothermie der Nitrierungsreaktion sind hierbei geeignete Sicherheitsmaßnahmen einzuhalten. Ein derartiges Herstellverfahren ist für andere Alkylnitrate in der US-A 4 479 905 beschrieben.

Ein für die oben beschriebene Nitrierung einzusetzendes verzweigtes Decanol ist beispielsweise 2-n-Propylheptanol. Ein möglicher Syntheseweg für 2-n-Propylheptanol ist die Kondensation von 1-Pentanol oder einer Mischung von Methylbutan-1-olen in Gegenwart von Alkalimetallhydroxid, z.B. Kaliumhydroxid, bei erhöhten Temperaturen.

Von besonderem Interesse für die vorliegende Erfindung ist eine spezielle Mischung aus den genannten verzweigten Decylnitraten, die eine neue Stoffmischung darstellt. Daher ist auch Gegenstand der vorliegenden Erfindung eine Mischung aus verzweigten Decylnitraten, welche als Komponenten
10 bis 99 Gew.-%, insbesondere 40 bis 97 Gew.-% 2-n-Propylheptylnitrat (Verbindung la),
1 bis 90 Gew.-%, insbesondere 3 bis 60 Gew.-% 2-n-Propyl-4-methylhexylnitrat und/oder 2-n-Propyl-5-methylhexylnitrat (Verbindung Ic bzw. le) und
0 bis 50 Gew.-%, insbesondere 0 bis 30 Gew.-% sonstige verzweigte Decylnitrate, insbesondere solche gemäß der Formel I, beispielsweise eine oder mehrere der Verbindungen Ib, Id, le, If, Ig oder Ih,
umfaßt, wobei die Summe der genannten Komponenten 100 Gew.-% ergibt. Die Anteile der einzelnen verzweigten Decylnitrate an einer solchen Mischung lassen sich mit übliche analytischen Methoden wie der Gaschromatographie leicht bestimmen.

Ein typischer Vertreter dieser Mischung ist 2-n-Propylheptylnitrat technischer Qualität, welches in der Regel 80 bis 95 Gew.-% der reinen Verbindung la, 5 bis 15 Gew.-% der reinen Verbindung(en) Ic und/oder le und 0 bis 10 Gew.-% sonstiger verzweigter Decylnitrate enthält, wobei die Summe aller verzweigten Decylnitrate 100 Gew.-% ergibt. Ein typischer Vertreter für die sonstigen verzweigten Decylnitrate ist 2-iso-Propylheptylnitrat (Verbindung Ib), welches auch in geringen Mengen mit den üblichen analytischen Methoden gut nachweisbar ist. Daneben kann diese Mischung herstellungsbedingt in geringen Mengen noch Nebenprodukte enthalten. Technisches 2-n-Propylheptanol und seine Herstellung als Vorstufe für dieses 2-n-Propylheptylnitrat technischer Qualität sind beispielsweise in der US-A 4 426 542 beschrieben.

Die Herstellung der genannten Mischung aus verzweigten Decylnitraten, insbesondere des oben beschriebenen technischen 2-n-Propylheptynitrates, erfolgt zweckmäßigerweise durch eine Synthesesequenz, die durch die folgenden Verfahrensschritte gekennzeichnet ist:
(a) Hydroformylierung von Butenen zu C₅-Aldehyden,
(b) Dimerisierung der C₅-Aldehyde durch Aldolkondensation zu den entsprechendenC₁₀-Acroleinderivaten,
(c) Hydrierung der C₁₀-Acroleinderivate zu den entsprechenden Decanolen und
(d) Nitrierung der Decanole mittels eines Gemisches aus Salpetersäure und Schwefelsäure zu der Mischung aus verzweigten Decylnitraten.

Der Verfahrensschritt (a) ist beispielsweise in der US-A 4 287 370 beschrieben. Durch Hydroformylierung eines C₄-Kohlenwasserstoffstromes, der als Hauptkomponente 1-Buten neben 2-Buten, Isobuten und gegebenenfalls Butanen enthält, mittels eines Rhodium-Katalysatorkomplexes erhält man durch dieses vorzugsweise eingesetzte Hydroformylierungsverfahren eine Mischung aus n-Valeraldehyd (n-Pentanal), iso-Valeraldehyd (2-Methylbutanal) und gegebenenfalls Nebenkomponenten wie 3-Methylbutanal. Je nach Einsatzstoff-Zusammensetzung und Reaktionsbedingungen schwankt das Gew.-Verhältnis von n- Valeraldehyd zu iso-Valeraldehyd, es liegt meist im Bereich von 8 : 1 bis 20 : 1.

Der Verfahrensschritt (b) ist beispielsweise in der US-A 5 434 313 beschrieben. Durch Aldol-Kondensation, vorzugsweise mittels wässrigem Alkalimetallhydroxid, erhält man durch dieses vorzugsweise eingesetzte Dimerisierungsverfahren aus dem im Verfahrensschritt (a) erhaltenen n-Valeraldehyd 2-n-Propyl-3-n-butylacrolein (2-n-Propyl-2-heptenal) und aus dem erhaltenen iso-Valeraldehyd 2-n-Propyl-3-sec.-butylacrolein (2-n-Propyl-4-methyl-2-hexenal). Durch eventuell in geringen Mengen mit vorliegende isomere C₅-Aldehyde können weitere isomere C₁₀-Acroleinderivate entstehen.

Im Verfahrensschritt (c) werden die im Verfahrensschritt (b) erhaltenen C₁₀-Acrolein-derivate nach üblichen Methoden mit Wasserstoff zu den entsprechenden verzweigten Decanolen hydriert. Man verwendet hierzu üblicherweise heterogene Hydrierungskatalysatoren auf beispielsweise Nickel-, Kobalt- oder Kupfer-Basis, z.B. Raney-Nickel oder Kobalt auf Kieselgur. Aus dem 2-n-Propyl-3-n-butylacrolein (2-n-Propyl-2-heptenal) entsteht hierbei 2-n-Propylheptanol und aus dem 2-n-Propyl-3-sec.-butylacrolein (2-n-Propyl-4-methyl-2-hexenal) 2-n-Propyl-4-methylhexanol.

Die Nitrierung der im Verfahrensschritt (c) erhaltenen verzweigten Decanole erfolgt im Verfahrensschritt (d) mittels eines Gemisches aus Salpetersäure und Schwefelsäure (üblicherweise als "Nitriersäure" bezeichnet). Man arbeitet vorzugsweise mit einem Gemisch aus ca. 65 gew.-%iger Salpetersäure und ca. 96 gew.-%iger Schwefelsäure im Vol.-Verhältnis von ca. 2:3 als Nitriersäure. Die Nitriersäure wird üblicherweise in hohem Überschuß gegenüber den zu nitrierenden verzweigten Decanolen eingesetzt, beispielweise im Mol-Verhältnis HNO₃ zu Decanole von 10:1 bis 25:1. Die Nitrierungsreaktion wird in der Regel bei Temperaturen von -10 bis + 25°C, vorzugsweise bei 0 bis +15°C unter ausreichender Kühlung durchgeführt. Wegen der starken Exothermie der Nitrierungsreaktion sind hierbei geeignete Sicherheitsmaßnahmen einzuhalten. Ein derartiges Herstellverfahren ist für andere Alkylnitrate in der US-A 4 479 905 beschrieben.

Da die genannten verzweigten Decylnitrate und insbesondere die genannte Mischung aus verzweigten Decylnitraten die gewünschte Wirkung in Kraftstoffen als Verbrennungsverbesserer oder Cetanzahlverbesserer zeigen, ist weiterhin Gegenstand der vorliegenden Erfindung ein additivierter Kraftstoff, welcher einen größeren Anteil eines Grundkraftstoffes, einen kleineren Anteil mindestens eines der genannten verzweigten Decylnitrate oder der genannten Mischung aus verzweigten Decylnitraten sowie gegebenenfalls weitere übliche Kraftstoffadditive in den hierfür üblichen Mengen umfaßt.

Der erfindungsgemäße additivierte Kraftstoff umfasst in der Regel 20 bis 2000 Gew.-ppm, insbesondere 100 bis 1500 Gew.-ppm, vor allem 250 bis 1200 Gew.-ppm an verzweigten Decylnitraten. Typische Dosierraten sind 500.Gew.-ppm oder 1000 Gew.-ppm.

Die erfindungsgemäßen verzweigten Decylnitrate können im Prinzip in allen Grundkraftstoffarten, beispielsweise in Ottokraftstoff, in Mitteldestillat-Kraftstoff, hier insbesondere in Dieselkraftstoff und Heizöl, in Turbinenkraftstoff oder in Kerosin, zur Verbesserung der Verbrennung zum Einsatz kommen.

In einer bevorzugten Ausführungsform umfasst der erfindungsgemäße additivierte Kraftstoff einen Ottokraftstoff als Grundkraftstoff. Hierbei kommen alle handelsüblichen Ottokraftstoffzusammensetzungen in Betracht. Als typischer Vertreter soll hier der marktübliche Eurosuper Grundkraftstoff gemäß EN 228 genannt werden. Weiterhin sind auch Ottokraftstoffzusammensetzungen der Spezifikation gemäß WO 00/47698 mögliche Einsatzgebiete für die vorliegende Erfindung.

In einer weiteren bevorzugten Ausführungsform umfasst der erfindungsgemäße additivierte Kraftstoff einen Mitteldestillat-Kraftstoff, insbesondere einen Dieselkraftstoff, als Grundkraftstoff. Bei Mitteldestillat-Kraftstoffen und Dieselkraftstoffen handelt es sich üblicherweise um Erdölraffinate, die in der Regel einen Siedebereich von 100 bis 400°C haben. Dies sind meist Destillate mit einem 95%-Punkt bis zu 360°C oder auch darüber hinaus. Dies können aber auch sogenannte "Ultra low sulfur diesel" oder "City diesel" sein, gekennzeichnet durch einen 95%-Punkt von beispielsweise maximal 345°C und einem Schwefelgehalt von maximal 0,005 Gew.-% oder durch einen 95%-Punkt von beispielsweise 285°C und einem Schwefelgehalt von maximal 0,001 Gew.-%. Neben den durch Raffination erhältlichen Dieselkraftstoffen, deren Hauptbestandteile längerkettige Paraffine darstellen, sind solche, die durch Kohlevergasung oder Gasverflüssigung ["gas to liquid" (GTL) Kraftstoffe] erhältlich sind, geeignet. Geeignet sind auch Mischungen der vorstehend genannten Dieselkraftstoffe mit regenerativen Kraftstoffen wie Biodiesel oder Bioethanol. Von besonderem Interesse sind gegenwärtig Dieselkraftstoffe mit niedrigem Schwefelgehalt, das heißt mit einem Schwefelgehalt von weniger als 0,05 Gew.-%, vorzugsweise von weniger als 0,02 Gew.-%, insbesondere von weniger als 0,005 Gew.-% und speziell von weniger als 0,001 Gew.-% Schwefel. Dieselkraftstoffe können auch Wasser, z.B. in einer Menge bis zu 20 Gew.-%, enthalten, beispielsweise in Form von Diesel-Wasser-Mikroemulsionen oder als sogenannter "White Diesel".

Weiterhin kann der erfindungsgemäße additivierte Kraftstoff auch ein Heizöl umfassen. Bei Heizölen handelt es sich beispielsweise um schwefelarme oder schwefelreiche Erdölraffinate oder um Stein- oder Braunkohledestillate, die üblicherweise einen Siedebereich von 150 bis 400°C aufweisen. Bei Heizölen kann es sich um Standard-Heizöl gemäß DIN 51603-1 handeln, das einen Schwefelgehalt von 0,005 bis 0,2 Gew.-% besitzt, oder es handelt sich um schwefelarme Heizöle mit einem Schwefelgehalt von 0 bis 0,005 Gew.-%. Als Beispiele für Heizöl sei insbesondere Heizöl für häusliche Ölfeuerungsanlagen oder Heizöl EL genannt.

Weiterhin kann der erfindungsgemäße additivierte Kraftstoff auch einen Turbinenkraftstoff umfassen. Hierbei handelt es sich beispielsweise um einen in der zivilen oder militärischen Luftfahrt üblichen flüssigen Turbinenkraftstoff. Dazu zählen beispielsweise Kraftstoffe der Bezeichnung Jet Fuel A, Jet Fuel A-1, Jet Fuel B, Jet Fuel JP-4, JP-5, JP-7, JP-8 und JP-8+100. Jet A und Jet A-1 sind kommerziell erhältliche Turbinenkraftstoffspezifikationen auf Kerosinbasis. Jet B ist ein weiter geschnittener Kraftstoff auf Basis von Naphtha- und Kerosinfraktionen. JP-4 ist äquivalent zu Jet B. JP-5, JP-7, JP-8 und JP-8+100 sind militärische Turbinenkraftstoffe, wie sie beispielsweise von der Marine und Luftwaffe eingesetzt werden. Zum Teil bezeichnen diese Normen Formulierungen, die bereits weitere Additive, wie Korrosionsinhibitoren, Vereisungsinhibitoren, statische Dissipatoren, etc. enthalten.

Die erfindungsgemäßen verzweigten Decylnitrate oder die erfindungsgemäße Mischung aus verzweigten Decylnitraten können entweder dem Grundkraftstoff, insbesondere dem Dieselkraftstoff, allein oder in Form von Diesel-Performance-Paketen zugesetzt werden. Derartige Diesel-Performance-Pakete stellen Kraftstoffadditiv-Konzentrate dar und enthalten in der Regel neben Lösungsmitteln noch eine Reihe weiterer Komponenten als Coadditive, beispielsweise Trägeröle, Kaltfließverbesserer, Korrosionsinhibitoren, Demulgatoren, Dehazer, Antischaummittel, weitere Cetanzahlverbesserer, weitere Verbrennungsverbesserer, Antioxidantien oder Stabilisatoren, Antistatika, Metallocene, Metalldeaktivatoren, Lösungsvermittler, Marker und/oder Farbstoffe.

In einer bevorzugten Ausführungsform umfasst der erfindungsgemäße additivierte Kraftstoff, hier insbesondere Ottokraftstoffe und Dieselkraftstoffe, neben den erfindungsgemäßen verzweigten Decylnitraten oder der erfindungsgemäßen Mischung aus verzweigten Decylnitraten, die nachfolgend als Komponente (A) bezeichnet werden, als weitere Kraftstoffadditive mindestens ein Detergens, nachfolgend als Komponente (B) bezeichnet.

Als Detergentien oder Detergens-Additive (B) werden üblicherweise Ablagerungsinhibitoren für Kraftstoffe bezeichnet. Vorzugsweise handelt es sich bei den Detergentien um amphiphile Substanzen, die mindestens einen hydrophoben Kohlenwasserstoffrest mit einem zahlengemittelten Molekulargewicht (Mₙ) von 85 bis 20.000, insbesondere von 300 bis 5000, vor allem von 500 bis 2500, und mindestens eine polare Gruppierung besitzen, die ausgewählt ist unter
(Ba) Mono- oder Polyaminogruppen mit bis zu 6 Stickstoffatomen, wobei mindestens ein Stickstoffatom basische Eigenschaften hat;
(Bb) Nitrogruppen, gegebenenfalls in Kombination mit Hydroxylgruppen;
(Bc) Hydroxylgruppen in Kombination mit Mono- oder Polyaminogruppen, wobei mindestens ein Stickstoffatom basische Eigenschaften hat;
(Bd) Carboxylgruppen oder deren Alkalimetall- oder Erdalkalimetallsalzen;
(Be) Sulfonsäuregruppen oder deren Alkalimetall- oder Erdalkalimetallsalzen;
(Bf) Polyoxy-C₂-C₄-alkylengruppierungen, die durch Hydroxylgruppen, Mono- oder Polyaminogruppen, wobei mindestens ein Stickstoffatom basische Eigenschaften hat, oder durch Carbamatgruppen terminiert sind;
(Bg) Carbonsäureestergruppen;
(Bh) aus Bernsteinsäureanhydrid abgeleiteten Gruppierungen mit Hydroxy- und/oder Amino- und/oder Amido- und/oder Imidogruppen; und/oder
(Bi) durch Mannich-Umsetzung von substituierten Phenolen mit Aldehyden und Mono- oder Polyaminen erzeugten Gruppierungen.

Der hydrophobe Kohlenwasserstoffrest in den obigen Detergens-Additiven, welcher für die ausreichende Löslichkeit in der Brennstoffölzusammensetzung sorgt, hat ein zahlengemitteltes Molekulargewicht (Mₙ) von 85 bis 20.000, insbesondere von 300 bis 5000, vor allem von 500 bis 2500. Als typischer hydrophober Kohlenwasserstoffrest, insbesondere in Verbindung mit den polaren Gruppierungen (Ba), (Bc), (Bh) und (Bi), kommen längerkettige Alkyl- oder Alkenylgruppen, insbesondere der Polypropenyl-, Polybutenyl- und Polyisobutenylrest mit jeweils Mₙ = 300 bis 5000, insbesondere 500 bis 2500, vor allem 700 bis 2300, in Betracht.

Als Beispiele für obige Gruppen von Detergens-Additiven seien die folgenden genannt:

Mono- oder Polyaminogruppen (Ba) enthaltende Additive sind vorzugsweise Polyalkenmono- oder Polyalkenpolyamine auf Basis von Polypropen oder konventionellem (d.h. mit überwiegend mittenständigen Doppelbindungen) Polybuten oder Polyisobuten mit Mₙ = 300 bis 5000. Geht man bei der Herstellung der Additive von Polybuten oder Polyisobuten mit überwiegend mittenständigen Doppelbindungen (meist in der β- und γ-Position) aus, bietet sich der Herstellweg durch Chlorierung und anschließende Aminierung oder durch Oxidation der Doppelbindung mit Luft oder Ozon zur Carbonyl- oder Carboxylverbindung und anschließende Aminierung unter reduktiven (hydrierenden) Bedingungen an. Zur Aminierung können hier Amine, wie z. B. Ammoniak, Monoamine oder Polyamine, wie Dimethylaminopropylamin, Ethylendiamin, Diethylentriamin, Triethylentetramin oder Tetraethylenpentamin, eingesetzt werden. Entsprechende Additive auf Basis von Polypropen sind insbesondere in der WO-A-94/24231 beschrieben.

Weitere bevorzugte Monoaminogruppen (Ba) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in WO-A-97/03946 beschrieben sind.

Weitere bevorzugte Monoaminogruppen (Ba) enthaltende Additive sind die aus Polyisobutenepoxiden durch Umsetzung mit Aminen und nachfolgender Dehydratisierung und Reduktion der Aminoalkohole erhältlichen Verbindungen, wie sie insbesondere in DE-A-196 20 262 beschrieben sind.

Nitrogruppen (Bb), gegebenenfalls in Kombination mit Hydroxylgruppen, enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in WO-A-96/03367 und WO-A-96/03479 beschrieben sind. Diese Umsetzungsprodukte stellen in der Regel Mischungen aus reinen Nitropolyisobutenen (z. B. α,β-Dinitropolyisobuten) und gemischten Hydroxynitropolyisobutenen (z. B. α-Nitro-β-hydroxypolyisobuten) dar.

Hydroxylgruppen in Kombination mit Mono- oder Polyaminogruppen (Bc) enthaltende Additive sind insbesondere Umsetzungsprodukte von Polyisobutenepoxiden, erhältlich aus vorzugsweise überwiegend endständige Doppelbindungen aufweisendem Polyisobuten mit Mₙ = 300 bis 5000, mit Ammoniak, Mono- oder Polyaminen, wie sie insbesondere in EP-A-476 485 beschrieben sind.

Carboxylgruppen oder deren Alkalimetall- oder Erdalkalimetallsalze (Bd) enthaltende Additive sind vorzugsweise Copolymere von C₂-C₄₀-Olefinen mit Maleinsäureanhydrid mit einer Gesamt-Molmasse von 500 bis 20000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der EP-A-307 815 bekannt. Derartige Additive dienen hauptsächlich zur Verhinderung von Ventilsitzverschleiß und können, wie in der WO-A-87/01126 beschrieben, mit Vorteil in Kombination mit üblichen Kraftstoffdetergenzien wie Poly(iso)-butenaminen oder Polyetheraminen eingesetzt werden.

Sulfonsäuregruppen oder deren Alkalimetall- oder Erdalkalimetallsalze (Be) enthaltende Additive sind vorzugsweise Alkalimetall- oder Erdalkalimetallsalze eines Sulfobernsteinsäurealkylesters, wie er insbesondere in der EP-A-639 632 beschrieben ist. Derartige Additive dienen hauptsächlich zur Verhinderung von Ventilsitzverschleiß und können mit Vorteil in Kombination mit üblichen Kraftstoffdetergenzien wie Poly(iso)-butenaminen oder Polyetheraminen eingesetzt werden.

Polyoxy-C₂-C₄-alkylengruppierungen (Bf) enthaltende Additive sind vorzugsweise Polyether oder Polyetheramine, welche durch Umsetzung von C₂-C₆₀-Alkanolen, C₆-C₃₀-Alkandiolen, Mono- oder Di-C₂-C₃₀-alkylaminen, C₁-C₃₀-Alkylcyclohexanolen oder C₁-C₃₀-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in EP-A-310 875, EP-A-356 725, EP-A-700 985 und US-A-4 877 416 beschrieben. Im Falle von Polyethern erfüllen solche Produkte auch Trägeröleigenschaften. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Carbonsäureestergruppen (Bg) enthaltende Additive sind vorzugsweise Ester aus Mono-, Di- oder Tricarbonsäuren mit langkettigen Alkanolen oder Polyolen, insbesondere solche mit einer Mindestviskosität von 2 mm²/s bei 100°C, wie sie insbesondere in DE-A-38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tridecanols. Derartige Produkte erfüllen auch Trägeröleigenschaften.

Aus Bernsteinsäureanhydrid abgeleitete Gruppierungen mit Hydroxy- und/oder Amino- und/oder Amido- und/oder Imidogruppen (Bh) enthaltende Additive sind vorzugsweise entsprechende Derivate von Alkyl- oder Alkenyl-substituiertem Bernsteinsäureanhydrid und insbesondere die entsprechenden Derivate von Polyisobutenylbernsteinsäureanhydrid, welche durch Umsetzung von konventionellem oder hochreaktivem Polyisobuten mit Mₙ = 300 bis 5000 mit Maleinsäureanhydrid auf thermischem Weg oder über das chlorierte Polyisobuten erhältlich sind. Von besonderem Interesse sind hierbei Derivate mit aliphatischen Polyaminen wie Ethylendiamin, Diethylentriamin, Triethylentetramin oder Tetraethylenpentamin. Bei den Gruppierungen mit Hydroxy- und/oder Amino- und/oder Amido- und/oder Imidogruppen handelt es sich beispielsweise um Carbonsäuregruppen, Säureamide von Monoaminen, Säureamide von Di- oder Polyaminen, die neben der Amidfunktion noch freie Amingruppen aufweisen, Bernsteinsäurederivate mit einer Säure- und einer Amidfunktion, Carbonsäureimide mit Monoaminen, Carbonsäureimide mit Di- oder Polyaminen, die neben der Imidfunktion noch freie Amingruppen aufweisen, oder Diimide, die durch die Umsetzung von Di- oder Polyaminen mit zwei Bernsteinsäurederivaten gebildet werden. Derartige Kraftstoffadditive sind insbesondere in US-A-4 849 572 beschrieben.

Durch Mannich-Umsetzung von substituierten Phenolen mit Aldehyden und Mono- oder Polyaminen erzeugte Gruppierungen (Bi) enthaltende Additive sind vorzugsweise Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin oder Dimethylaminopropylamin. Die Polyisobutenyl-substituierten Phenole können aus konventionellem oder hochreaktivem Polyisobuten mit Mₙ = 300 bis 5000 stammen. Derartige "Polyisobuten-Mannichbasen" sind insbesondere in der EP-A-831 141 beschrieben.

Zur genaueren Definition der einzelnen aufgeführten Kraftstoffadditive wird hier auf die Offenbarungen der oben genannten Schriften des Stands der Technik ausdrücklich Bezug genommen.

Besonders bevorzugt sind Detergens-Additive aus der Gruppe (Bh). Hierbei handelt es sich vorzugsweise um die Umsetzungsprodukte von Alkyl- oder Alkenyl-substituierten Bernsteinsäureanhydriden, insbesondere von Polyisobutenylbernsteinsäureanhydriden, mit Aminen und/oder Alkoholen. Es handelt sich hierbei somit um von Alkyl-, Alkenyl- oder Polyisobutenyl-Bernsteinsäureanhydrid abgeleitete Derivate mit Amino- und/oder Amido- und/oder Imido- und/oder Hydroxylgruppen. Es versteht sich von selbst, dass diese Umsetzungsprodukte nicht nur bei Einsatz von substituiertem Bernsteinsäureanhydrid, sondern auch bei Verwendung von substituierter Bernsteinsäure oder geeigneten Säurederivaten, wie Bernsteinsäurehalogenide oder -ester, erhältlich sind.

In einer besonders bevorzugten Ausführungsform umfasst der erfindungsgemäße additivierte Kraftstoff als weitere Kraftstoffadditive mindestens ein Detergens auf Basis eines Polyisobutenyl-substituierten Bernsteinsäureimids. Speziell von Interesse sind die Imide mit aliphatischen Polyaminen. Besonders bevorzugte Polyamine sind dabei Ethylendiamin, Diethylentriamin, Triethylentetramin, Pentaethylenhexamin und vor allem Tetraethylenpentamin. Der Polyisobutenylrest besitzt ein zahlenmittleres Molekulargewicht Mₙ von vorzugsweise 500 bis 5000, besonders bevorzugt von 500 bis 2000 und insbesondere von etwa 1000.

Verzugsweise werden die genannten Detergens-Additive (B) zusammen mit der Komponente (A) in Kombination mit wenigstens einem Trägeröl verwendet.

Geeignete mineralische Trägeröle sind bei der Erdölverarbeitung anfallende Fraktionen, wie Brightstock oder Grundöle mit Viskositäten wie beispielsweise aus der Klasse SN 500 - 2000; aber auch aromatische Kohlenwasserstoffe, paraffinische Kohlenwasserstoffe und Alkoxyalkanole. Brauchbar ist ebenfalls eine als "hydrocrack oil" bekannte und bei der Raffination von Mineralöl anfallende Fraktion (Vakuumdestillatschnitt mit einem Siedebereich von etwa 360 bis 500°C, erhältlich aus unter Hochdruck katalytisch hydriertem und isomerisiertem sowie entparaffiniertem natürlichen Mineralöl). Ebenfalls geeignet sind Mischungen oben genannter mineralischer Trägeröle.

Beispiele für geeignete synthetische Trägeröle sind ausgewählt unter: Polyolefinen (Polyalphaolefinen oder Polyinternalolefinen), (Poly)estern, (Poly)alkoxylaten, Polyethern, aliphatischen Polyetheraminen, alkylphenolgestarteten Polyethern, alkylphenolgestarteten Polyetheraminen und Carbonsäureestern langkettiger Alkanole.

Beispiele für geeignete Polyolefine sind Olefinpolymerisate mit Mₙ = 400 bis 1800, vor allem auf Polybuten- oder Polyisobuten-Basis (hydriert oder nicht hydriert).

Beispiele für geeignete Polyether oder Polyetheramine sind vorzugsweise Polyoxy-C₂-C₄-alkylengruppierungen enthaltende Verbindungen, welche durch Umsetzung von C₂-C₆₀-Alkanolen, C₆-C₃₀-Alkandiolen, Mono- oder Di-C₂-C₃₀-alkylaminen, C₁-C₃₀-Alkyl-cyclohexanolen oder C₁-C₃₀-Alkylphenolen mit 1 bis 30 mol Ethylenoxid und/ oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in EP-A 310 875, EP-A 356 725, EP-A 700 985 und US-A 4,877,416 beschrieben. Beispielsweise können als Polyetheramine Poly-C₂-C₆-Alkylenoxidamine oder funktionelle Derivate davon verwendet werden. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Beispiele für Carbonsäureester langkettiger Alkanole sind insbesondere Ester aus Mono-, Di- oder Tricarbonsäuren mit langkettigen Alkanolen oder Polyolen , wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des Isooctanols, Isononanols, Isodecanols und des Isotridecanols, wie z. B. Di-(n- oder iso-tridecyl)phthalat.

Weitere geeignete Trägerölsysteme sind beispielsweise in DE-A 38 26 608, DE-A 41 42 241, DE-A 43 09 074, EP-A 0 452 328 und EP-A 0 548 617 beschrieben. Beispiele für besonders geeignete synthetische Trägeröle sind alkoholgestartete Polyether mit etwa 5 bis 35, wie z. B. etwa 5 bis 30, C₃-C₆-Alkylenoxideinheiten, wie z. B. ausgewählt unter Propylenoxid-, n-Butylenoxid- und i-Butylenoxid-Einheiten, oder Gemischen davon. Nichtlimitierende Beispiele für geeignete Starteralkohole sind langkettige Alkanole oder mit langkettigem Alkyl-substituierte Phenole, wobei der langkettige Alkylrest insbesondere für einen geradkettigen oder verzweigten C₆-C₁₈-Alkylrest steht. Als bevorzugte Beispiele sind zu nennen Tridecanol und Nonylphenol.

Weitere geeignete synthetische Trägeröle sind alkoxylierte Alkylphenole, wie sie in der DE-A 101 02 913 beschrieben sind.

Bevorzugte Trägeröle sind synthetische Trägeröle, wobei Polyether besonders bevorzugt sind.

Dem erfindungsgemäßen additivierten Kraftstoff wird das Detergens-Additiv (B) oder ein Gemisch verschiedener solcher Detergens-Additive in einer Gesamtmenge von vorzugsweise 10 bis 2000 Gew.-ppm, besonders bevorzugt von 20 bis 1000 Gew.-ppm, stärker bevorzugt von 50 bis 500 Gew.-ppm und insbesondere von 50 bis 200 Gew.-ppm, z.B. von 70 bis 150 Gew.-ppm, zugesetzt.

Wenn ein Trägeröl mitverwendet wird, so wird dieses dem erfindungsgemäßen additivierten Kraftstoff in einer Menge von vorzugsweise 1 bis 1000 Gew.-ppm, besonders bevorzugt von 10 bis 500 Gew.-ppm und insbesondere von 20 bis 100 Gew.-ppm, zugesetzt.

Als weitere Coadditive geeignete Kaltfließverbesserer sind beispielsweise Copolymere von Ethylen mit wenigstens einem weiteren ungesättigten Monomer, z.B. EthylenVinylacetat-Copolymere.

Als weitere Coadditive geeignete Korrosionsinhibitoren sind beispielsweise Bernsteinsäureester, vor allem mit Polyolen, Fettsäurederivate, z.B. Ölsäureester, oligomerisierte Fettsäuren und substituierte Ethanolamine.

Als weitere Coadditive geeignete Demulgatoren sind beispielsweise die Alkali- und Erdalkalimetallsalze von alkylsubstituierten Phenol- und Naphthalinsulfonaten und die Alkali- und Erdalkalimetallsalze von Fettsäure, weiterhin Alkoholalkoxylate, z.B. Alkoholethoxylate, Phenolalkoxylate, z.B. tert.-Butylphenolethoxylate oder tert.-Pentylphenolethoxylate, Fettsäure, Alkylphenole, Kondensationsprodukte von Ethylenoxid und Propylenoxid, z.B. Ethylenoxid-Propylenoxid-Blockcopolymere, Polyethylenimine und Polysiloxane.

Als weitere Coadditive geeignete Dehazer sind beispielsweise alkoxylierte Phenol-Formaldehyd-Kondensate.

Als weitere Coadditive geeignete Antischaummittel sind beispielsweise Polyethermodifizierte Polysiloxane.

Als weitere Coadditive geeignete Cetanzahl- und Verbrennungsverbesserer sind beispielsweise von den erfindungsgemäßen verzweigten Decylnitraten verschiedene Alkylnitrate, z.B. Cyclohexylnitrat und insbesondere 2-Ethylhexylnitrat, und Peroxide, z.B. Di-tert.-butylperoxid.

Als weitere Coadditive geeignete Antioxidantien sind beispielsweise substituierte Phenole, z.B. 2,6-Di-tert.-butylphenol und 2,6-Di-tert.-butyl-3-methylphenol, sowie Phenylendiamine, z.B. N,N'-Di-sec.-butyl-p-phenylendiamin.

Als weitere Coadditive geeignete Metalldeakivatoren sind beispielsweise Salicylsäure-Derivate, z.B. N,N'-Disalicyliden-1,2-propandiamin.

Als Lösungsmittel eignen sich, insbesondere für Diesel-Performance Pakete, beispielsweise unpolare organische Lösungsmittel, insbesondere aromatische und aliphatische Kohlenwasserstoffe, z.B. Toluol, Xylole, "white spirit" sowie die technischen Lösungsmittelgemische der Bezeichnungen Shellsol® (Hersteller: Royal Dutch / Shell Group), Exxol® (Hersteller: ExxonMobil) und Solvent Naphtha. Weiterhin kommen hier, insbesondere in Abmischung mit den genannten unpolaren organischen Lösungsmitteln, polare organische Lösungsmittel, vor allem Alkohole wie 2-Ethylhexanol, Decanol und Isotridecanol, in Betracht.

Wenn die genannten Coadditive und/oder Lösungsmittel mitverwendet werden, werden sie in den hierfür übliche Mengen eingesetzt.

Gegenstand der vorliegenden Erfindung ist auch ein Kraftstoffadditiv-Konzentrat, beispielsweise ein Diesel-Performance-Paket, welches, jeweils bezogen auf die Gesamtmenge des Kraftstoffadditiv-Konzentrates,
(A) 0,5 bis 80 Gew.-%, insbesondere 5 bis 75 Gew.-%, vor allem 10 bis 70 Gew.-%, mindestens eines erfindungsgemäßen verzweigten Decylnitrates oder einer erfindungsgemäßen Mischung aus verzweigten Decylnitraten sowie
(B) 0,5 bis 60 Gew.-%, insbesondere 3 bis 55 Gew.-%, vor allem 5 bis 50 Gew.-%, mindestens eines Detergenzes, insbesondere mindestens eines Detergenzes auf Basis eines Polyisobutenyl-substituierten Bernsteinsäureimids; enthält.

Darüber hinaus enthält das erfindungsgemäße Kraftstoffadditiv-Konzentrat in der Regel eines oder mehrere der weiteren oben genannten Coadditive und/oder der oben genannten Lösungsmittel.

Gegenstand der vorliegenden Erfindung ist weiterhin die Verwendung der erfindungsgemäßen verzweigten Decylnitrate oder der erfindungsgemäßen Mischung aus verzweigten Decylnitraten als Verbrennungsverbesserer und/oder Cetanzahlverbesserer in Kraftstoffen.

Die erfindungsgemäßen verzweigten Decylnitrate und die erfindungsgemäße Mischung hieraus sind als Kraftstoffadditive in Hinblick auf die Erhöhung der Cetanzahl in Dieselkraftstoff mindestens genauso wirksam wie der Marktstandard 2-Ethylhexylnitrat. Im Vergleich zu 2-Ethylhexylnitrat weisen sie jedoch eine geringere Flüchtigkeit, einen höheren Flammpunkt und eine höhere Temperatur der autokatalytischen Zersetzung, d.h. eine bessere Thermostabilität, auf. Weiterhin besitzten sie auch einen geringeren prozentualen Stickstoffgehalt (2-Ethylhexylnitrat: 8,0 Gew.-% N, Decylnitrate: 6,9 Gew.-% N) und sorgen somit für einen geringeren Gehalt an Stickoxiden in den Auspuffgasen. Sie weisen eine gute Tieftemperatur-Performance, insbesondere einen geringen Pour Point, auf. Ferner wurde beobachtet, dass sie den im Laufe des Motorenbetriebes zunehmenden Partikelausstoß verringern, indem sie unter anderen Effekten auch die Bildung von Ablagerungen in den Einlaßsystemen und Einspritzsystemen (Injektoren) der Motore verringern oder verhindern. Dieser Effekt wird insbesondere bei direkteinspritzenden Dieselmotoren, speziell in Common-Rail-Einspritzsystemen, beobachtet.

Die nachfolgenden Beispiele sollen die vorliegende Erfindung erläutern, ohne sie zu begrenzen.

### Beispiel: Bestimmung von Cetanzahlen mit Dieselkraftstoff

Die Cetanzahlen des gleichen handelsüblichen Dieselkraftstoffes ohne Cetanzahlverbesser (Versuch 1), mit 2-Ethylhexylnitrat als Cetanzahlverbesserer (Versuche 2 und 4) und mit einem 2-n-Propylheptylnitrat technischer Qualität, welches 88 Gew.-% reines 2-n-Propylheptylnitrat, zusammen 11,5 Gew.-% 2-n-Propyl-4-methylhexylnitrat und 2-n-Propyl-5-methylhexylnitrat sowie 0,5 Gew.-% sonstige verzweigte Decylnitrate (hiervon 0,1 Gew.-% 2-iso-Propylheptylnitrat) enthielt (Versuche 3 und 5), wurden gemäß der Norm EN ISO 5165 in einem BASF-MWM-Motor gemäß DIN 51 773 bestimmt und gemäß EN 590 auf CFR-Niveau korrigiert. Die nachfolgende Tabelle zeigt die Ergebnisse:

| Versuch | Cetanzahlverbesserer | Dosierung | Ergebnis |
|---|---|---|---|
| Nr.1 | ohne | - | 50,3 |
| Nr. 2 | 2-Ethylhexylnitrat | 500 Gew.-ppm | 52,7 |
| Nr. 3 | 2-n-Propylheptylnitrat technischer Qualität | 500 Gew.-ppm | 52,0 |
| Nr. 4 | 2-Ethylhexylnitrat | 1000 Gew.-ppm | 53,4 |
| Nr. 5 | 2-n-Propylheptylnitrat technischer Qualität | 1000 Gew.-ppm | 53,4 |

Bei einer Dosierung von 500 Gew.-ppm Cetanzahlverbesserer liegt der Wert für das 2-n-Propylheptylnitrat technischer Qualität in der Größeordnung des Marktstandards 2-Ethylhexylnitrat, bei einer Dosierung von 1000 Gew.-ppm sind beide Werte identisch.

## Patentansprüche

1. Verzweigte Decylnitrate der Formel I
R¹R²CH-CH₂-O-NO₂ (I)
in der R¹ einen n-Propyl- oder iso-Propylrest bezeichnet und R² einen linearen oder verzweigten Alkylrest mit 5 Kohlenstoffatomen bedeutet.

2. Verfahren zur Herstellung von verzweigten Decylnitraten gemäß Anspruch 1, **dadurch gekennzeichnet, dass** man die entsprechenden verzweigten Decanole mittels eines Gemisches aus Salpetersäure und Schwefelsäure nitriert.

3. Mischung aus verzweigten Decylnitraten, umfassend als Komponenten
| | |
|---|---|
| 10 bis 99 Gew.-% | 2-n-Propylheptylnitrat, |
| 1 bis 90 Gew.-% | 2-n-Propyl-4-methylhexylnitrat und/oder 2-n-Propyl-5-methylhexylnitrat und |
| 0 bis 50 Gew.-% | sonstige verzweigte Decylnitrate gemäß der Formel I in Anspruch 1, |
wobei die Summe der genannten Komponenten 100 Gew.-% ergibt.

4. Verfahren zur Herstellung der Mischung aus verzweigten Decylnitraten gemäß Anspruch 3, **gekennzeichnet durch** die Verfahrensschritte
(A) Hydroformylierung von Butenen zu C₅-Aldehyden,
(B) Dimerisierung der C₅-Aldehyde **durch** Aldolkondensation zu den entsprechenden C₁₀-Acroleinderivaten,
(C) Hydrierung der C₁₀-Acroleinderivate zu den entsprechenden Decanolen und
(D) Nitrierung der Decanole mittels eines Gemisches aus Salpetersäure und Schwefelsäure zu der Mischung aus verzweigten Decylnitraten.

5. Additivierter Kraftstoff, umfassend einen größeren Anteil eines Grundkraftstoffes, einen kleineren Anteil mindestens eines verzweigten Decylnitrates gemäß Anspruch 1 oder einer Mischung aus verzweigten Decylnitraten gemäß Anspruch 3 sowie gegebenenfalls weitere übliche Kraftstoffadditive in den hierfür üblichen Mengen.

6. Additivierter Kraftstoff nach Anspruch 5, umfassend 20 bis 2000 Gew.-ppm an verzweigten Decylnitraten.

7. Additivierter Kraftstoff nach Anspruch 5 oder 6, umfassend einen Mitteldestillat-Kraftstoff als Grundkraftstoff.

8. Additivierter Kraftstoff nach Anspruch 5 oder 6, umfassend einen Ottokraftstoff als Grundkraftstoff.

9. Additivierter Kraftstoff nach den Ansprüchen 5 bis 8, umfassend als weitere Kraftstoffadditive mindestens ein Detergens auf Basis eines Polyisobutenylsubstituier-ten Bernsteinsäureimids.

10. Kraftstoffadditiv-Konzentrat, enthaltend, jeweils bezogen auf die Gesamtmenge des Kraftstoffadditiv-Konzentrates,
(A) 0,5 bis 80 Gew.-% mindestens eines verzweigten Decylnitrates gemäß Anspruch 1 oder einer Mischung aus verzweigten Decylnitraten gemäß Anspruch 3 sowie
(B) 0,5 bis 60 Gew.-% mindestens eines Detergenzes.

11. Verwendung von verzweigten Decylnitraten gemäß Anspruch 1 oder einer Mischung aus verzweigten Decylnitraten gemäß Anspruch 3 als Verbrennungsverbesserer und/oder Cetanzahlverbesserer in Kraftstoffen.

## Claims

1. A branched decyl nitrate of the formula I
R¹R²CH-CH₂-O-NO₂ (I)
in which R¹ is an n-propyl or isopropyl radical and R² is a linear or branched alkyl radical having 5 carbon atoms.

2. A process for preparing branched decyl nitrates according to claim 1, which comprises nitrating the corresponding branched decanols by means of a mixture of nitric acid and sulfuric acid.

3. A mixture of branched decyl nitrates comprising, as components,
| | |
|---|---|
| from 10 to 99% | by weight of 2-n-propylheptyl nitrate, |
| from 1 to 90% | by weight of 2-n-propyl-4-methylhexyl nitrate and/or 2-n-propyl-5-methylhexylnitrate and |
| from 0 to 50% | by weight of other branched decyl nitrates of the formula I in claim 1, |
where the sum of the components mentioned adds up to 100% by weight.

4. A process for preparing the mixture of branched decyl nitrates according to claim 3, **characterized by** the process steps of
(A) hydroformylating butenes to C₅-aldehydes,
(B) dimerizing the C₅-aldehydes by aldol condensation to give the corresponding C₁₀-acrolein derivates,
(C) hydrogenating the C₁₀-acrolein derivates to the corresponding decanols and
(D) nitrating the decanols by means of a mixture of nitric acid and sulfuric acid to give the mixture of branched decyl nitrates.

5. An additized fuel comprising a major proportion of a base fuel, a minor proportion of at least one branched decyl nitrate according to claim 1 or of a mixture of branched decyl nitrates according to claim 3, and also, if appropriate, further customary fuel additives in the amounts customary therefor.

6. The additized fuel according to claim 5, comprising from 20 to 2000 ppm by weight of branched decyl nitrates.

7. The additized fuel according to claim 5 or 6, comprising a middle distillate fuel as the base fuel.

8. The additized fuel according to claim 5 or 6, comprising a gasoline fuel as the base fuel.

9. The additized fuel according to claims 5 to 8, comprising, as further fuel additives, at least one detergent based on a polyisobutenyl-substituted succinimide.

10. A fuel additive concentrate comprising, based in each case on the total amount of the fuel additive concentrate,
(A) from 0.5 to 80% by weight of at least one branched decyl nitrate according to claim 1 or of a mixture of branched decyl nitrates according to claim 3 and
(B) from 0.5 to 60% by weight of at least one detergent.

11. The use of branched decyl nitrates according to claim 1 or of a mixture of branched decyl nitrates according to claim 3 as combustion improvers and/or cetane number improvers in fuels.

## Revendications

1. Décylnitrates ramifiés de formule I
*R¹R²CH - CH₂ -O - NO₂* *(I)*
où R¹ désigne un radical n-propyle ou iso-propyle et R² signifie un radical alkyle linéaire ou ramifié comprenant 5 atomes de carbone.

2. Procédé pour la préparation de décylnitrates ramifiés selon la revendication 1, **caractérisé en ce qu'**on nitre les décanols ramifiés correspondants au moyen d'un mélange d'acide nitrique et d'acide sulfurique.

3. Mélange de décylnitrates ramifiés, comprenant, comme composants
| | |
|---|---|
| 10 à 99% en poids | de 2-n-propylheptylnitrate, |
| 1 à 90% en poids | de 2-n-propyl-4-méthylhexylnitrate et/ou de 2-n-propyl-5-méthylhexylnitrate et |
| 0 à 50% en poids | d'autres décylnitrates ramifiés de formule I dans la revendication 1, |
la somme des composants mentionnés étant de 100% en poids.

4. Procédé pour la préparation du mélange de décylnitrates ramifiés selon la revendication 3, **caractérisé par** les étapes de procédé
(A) Hydroformylation de butènes en C₅-aldéhydes,
(B) dimérisation des C₅-aldéhydes par condensation aldol en dérivés de C₁₀-acroléine correspondants,
(C) hydrogénation des dérivés de C₁₀-acroléine en décanols correspondants et
(D) nitration des décanols au moyen d'un mélange d'acide nitrique et d'acide sulfurique en mélange de décylnitrates ramifiés.

5. Carburant contenant des additifs, comprenant une proportion importante d'un carburant de base, une proportion plus petite d'au moins un décylnitrate ramifié selon la revendication 1 ou un mélange de décylnitrates ramifiés selon la revendication 3 ainsi que le cas échéant d'autres additifs usuels pour carburants aux quantités usuelles à cette fin.

6. Carburant contenant des additifs selon la revendication 5, comprenant 20 à 2000 ppm en poids de décylnitrates ramifiés.

7. Carburant contenant des additifs selon la revendication 5 ou 6, comprenant un carburant de distillat moyen comme carburant de base.

8. Carburant contenant des additifs selon la revendication 5 ou 6, comprenant de l'essence auto comme carburant de base.

9. Carburant contenant des additifs selon les revendications 5 à 8, comprenant comme autres additifs pour carburants un détergent à base d'un imide d'acide succinique substitué par polyisobutényle.

10. Concentrat d'additifs pour carburants contenant, à chaque fois par rapport à la quantité totale du concentrat d'additifs pour carburants,
(A) 0,5 à 80% en poids d'au moins un décylnitrate ramifié selon la revendication 1 ou d'un mélange de décylnitrates ramifiés selon la revendication 3, ainsi que
(B) 0,5 à 60% en poids d'au moins un détergent.

11. Utilisation de décylnitrates ramifiés selon la revendication 1 ou d'un mélange de décylnitrates ramifiés selon la revendication 3 comme agent d'amélioration de la combustion et/ou agent d'amélioration de l'indice de cétane dans les carburants.
